# FASCICULE DE BREVET EUROPEEN

(11) **EP 3 307 896 B1**
(45) Date de publication et mention de la délivrance du brevet: **07.06.2023**
(21) Numéro de dépôt: 16741068.7
(22) Date de dépôt: 10.06.2016
(51) Int. Cl.: C02F 103/20, C02F 103/26, C02F 3/28, C12P 5/02

(54) **NOUVEAU PROCÉDÉ DE MÉTHANISATION PAR VOIE SÈCHE**
TROCKENMETHANISIERUNGSVERFAHREN
DRY METHANISATION PROCESS

(30) Priorité: 12.06.2015 FR 1555339
(43) Date de publication de la demande: 18.04.2018
(73) Titulaire: SBM Developpement, 69130 Ecully (FR)
(72) Inventeur: JARDEL, Denis, 14950 Saint Etienne la Thillaye (FR); FRIMA, Henri, 01600 Saint Bernard (FR); BENBRAHIM, Mohammed, 68230 Turckheim (FR)
(74) Mandataire: Germain Maureau
(86) Numéro de dépôt international: PCT/FR2016/051398
(87) Numéro de publication internationale: WO 2016/198798

(56) Documents cités:
- EP-A1- 1 577 269
- EP-A2- 0 173 340
- DE-A1-102009 050 867
- MONTALVO S ET AL: "Effect of particle size and doses of zeolite addition on anaerobic digestion processes of synthetic and piggery wastes", PROCESS BIOCHEMISTRY, ELSEVIER, NL, vol. 40, no. 3-4, 1 mars 2005 (2005-03-01) , pages 1475-1481, XP027793785, ISSN: 1359-5113 [extrait le 2005-03-01]
- TADA C ET AL: "Effect of natural zeolite on methane production for anaerobic digestion of ammonium rich organic sludge", BIORESOURCE TECHNOLOGY, ELSEVIER BV, GB, vol. 96, no. 4, 1 mars 2005 (2005-03-01), pages 459-464, XP027800697, ISSN: 0960-8524 [extrait le 2005-03-01]
- SILVIO MONTALVO ET AL: "Application of natural zeolites in anaerobic digestion processes: A review", APPLIED CLAY SCIENCE, ELSEVIER SCIENCE, NL, vol. 58, 23 janvier 2012 (2012-01-23), pages 125-133, XP028473825, ISSN: 0169-1317, DOI: 10.1016/J.CLAY.2012.01.013 [extrait le 2012-01-30] cité dans la demande

## Description

La présente invention a pour objet un nouveau procédé de méthanisation par voie sèche.

La méthanisation (ou digestion anaérobie) est un processus naturel biologique de dégradation de la matière organique en absence d'oxygène. Il se produit naturellement dans certains sédiments, les marais, les rizières, ainsi que dans le tractus digestif de certains animaux.

Ce processus biologique forme un résidu de digestion stabilisé, appelé digestat, ainsi qu'un biogaz de composition variable contenant :
- entre 50 à 80 % de méthane (CH₄) ;
- entre 30 et 50 % de dioxyde de carbone (CO₂) ;
- de l'eau (H₂O) à saturation ;
- des composés traces (NH₃, H₂S, N₂, CO).

Le processus de méthanisation est complexe. La méthanisation est opérée par plusieurs populations microbiennes qui assurent les différentes phases (hydrolyse, acidogenèse, acétogenèse et méthanisation) de la biodégradation anaérobique de la matière organique. Ces populations microbiennes, présentes dans les substrats, sont très diverses et peuvent être stimulées par des additifs organiques ou minéraux. Au moins quatre groupes de micro-organismes sont ainsi impliqués dans le processus : des bactéries primaires fermentatives, des bactéries secondaires fermentatives ainsi que deux groupes de méthanogènes.

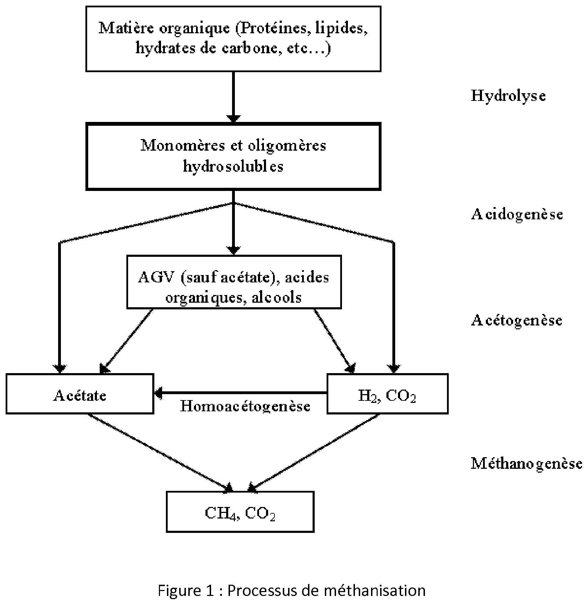

Le processus de méthanisation peut être subdivisé en quatre étapes biochimiques (Figure 1) :
- l'hydrolyse, durant laquelle les biopolymères (protéines, lipides, hydrates de carbone,...) sont hydrolysés en monomères et oligomères hydrosolubles grâce à des enzymes extracellulaires excrétées par des micro-organismes ;
- l'acidogenèse qui est réalisée par des bactéries dites acidogènes, durant laquelle les produits de l'hydrolyse sont transformés en acides gras volatils (AGV) tels que l'acétate, le propionate ou le butyrate ; en acides organiques tels que l'acide lactique ou l'acide succinique ; en hydrogène et en CO₂ ;
- l'acétogenèse, qui transforme les produits de l'acidogenèse (sauf l'acétate) en acétate. Elle se déroule suivant deux métabolismes : les acétogènes qui transforment les acides organiques en acétate, CO₂ et H₂ ; et les homoacétogènes qui combinent l'hydrogène et le CO₂ en acétate ;
- la méthanogenèse, dernière étape du processus de dégradation, forme le méthane suivant deux métabolismes : les méthanogènes acétotrophes transforment l'acétate en méthane et CO₂, alors que les méthanogenèse hydrogénotrophes combinent hydrogène et CO₂ pour former du méthane et de l'eau.

Chaque composé formé lors d'une de ces étapes biochimiques correspond au substrat utilisé lors de l'étape suivante. Cela forme donc une chaîne trophique dont le schéma est présenté en Figure 1.

Si l'enchainement des réactions biologiques est connu, la performance de chacune d'elles présente un caractère aléatoire dépendant de facteurs actuellement non totalement identifiés ni maîtrisés.

La maîtrise du processus de méthanisation permet de produire du méthane à partir de déchets ou de cultures. A l'échelle industrielle, la production de biogaz par méthanisation connait un développement industriel important, notamment en Europe, notamment en vue de répondre à des besoins spécifiques comme la production de gaz utilisable comme énergie à partir de déchets organiques (solides ou liquides).

La méthanisation, en tant que bioprocédé, peut être mise en oeuvre dans un digesteur, pour dépolluer des rejets chargés en matière organique tout en produisant de l'énergie sous forme de méthane. La méthanisation permet de traiter des rejets aussi divers que les eaux usées, les boues de stations d'épuration, les déjections animales, les déchets de l'industrie agro-alimentaire, les déchets de cuisine, les ordures ménagères ou encore les déchets agricoles. La méthanisation avec valorisation du biogaz produit (production d'énergie thermique et/ou électrique par combustion directe du méthane ou dans des moteurs thermiques) a toute sa place parmi l'ensemble des diverses solutions de production d'énergie renouvelable en permettant d'atteindre trois objectifs complémentaires : produire de l'énergie, réduire la charge polluante des déchets et des effluents et également, selon la nature du produit de départ, produire un digestat stabilisé utilisable comme fertilisant, amendement organique ou engrais.

La méthanisation des déchets solides s'applique à la plupart des déchets organiques. Cette technique s'applique à la fraction fermentescible des déchets qui doit être triée et recueillie par une collecte séparative, avant d'être méthanisée. Selon la provenance, on distingue différents types de déchets :
- les déchets municipaux tels que déchets alimentaires, journaux, emballages, textiles, déchets verts ou sous-produits de l'assainissement urbain ;
- les déchets industriels tels que les boues des industries agroalimentaires, les déchets de transformation des industries végétales et animales ou la fraction fermentescible des déchets industriels banals (DIB) ;
- les déchets agricoles tels que les déjections d'animaux, les substrats végétaux solides ou les bois déchiquetés ;
- les déchets littoraux tels que les algues vertes.

La méthanisation peut également être appliquée à des cultures dédiées telles que le maïs.

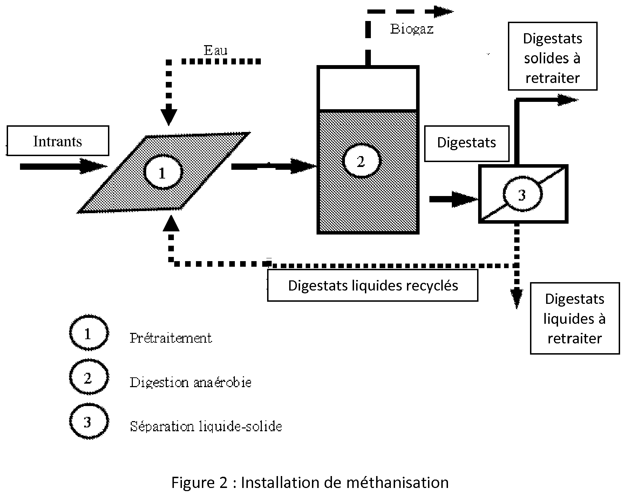

Il existe un grand nombre de procédés industriels de méthanisation. Leur fonctionnement peut être de type continu, discontinu ou semi-continu. Les procédés diffèrent également en fonction des types de prétraitements, des régimes de température auxquels ils fonctionnent, des types d'agitation et des post-traitements. Cependant, le schéma général de fonctionnement demeure identique, tel qu'illustré par la Figure 2.

Entre l'entrée et la sortie de l'installation de méthanisation, la matière va subir des étapes successives de prétraitements (1), de digestion (2) et de séparation liquide-solide (3) (Figure 2). Le flux de matières organiques va donc subir une transformation qui va finalement amener à trois types de produits : liquide, solide et gazeux. Dans la plupart des installations industrielles récentes, et notamment dans les installations fonctionnant par voie sèche, la séparation liquide-solide peut être suivie d'une étape de post-traitement des résidus de digestion, par exemple un séchage en utilisant les calories générées par la cogénération.

A ce jour, les installations existantes pour la mise en oeuvre de la méthanisation, notamment de la méthanisation « solide » ou « par voie sèche », restent en limite du seuil de rentabilité économique.

La production de biogaz par les procédés dits « voie sèche » est en effet affectée par des facteurs multiples et en particulier la nature des intrants saisonniers et l'inhibition de la méthanisation en phase anaérobie par la production en excès de composés perturbateurs tels que l'ammoniac ou les acides gras volatils (AGV).

Une réflexion est engagée pour rechercher des moyens de régulariser le processus de méthanisation soit en agissant sur l'évitement de la production d'excès de composés perturbateurs, en régulant ainsi le milieu réactionnel et en maintenant une production optimale de biogaz ; soit en favorisant/stimulant le développement et la multiplication de micro-organismes permettant d'augmenter les activités microbiennes dans le substrat et par conséquent la production de biogaz.

Cette réflexion intègre également la nécessité d'un équilibre économique favorable entre le coût des moyens d'amélioration à mettre en oeuvre et le résultat sur la production de biogaz, principale source de revenus, avec éventuellement l'amélioration de la valeur agronomique des digestats qui sont ensuite réutilisés en agriculture pour apporter des matières organiques aux sols et des éléments nutritifs aux cultures.

L'utilisation de zéolite pour augmenter la production de méthane dans le cadre de méthanisation par voie humide a déjà été étudiée.

Ainsi, différents auteurs ont rapporté l'utilisation de zéolite au cours du processus de méthanisation des eaux usées, en particulier les lisiers de porcs.

Dans « The impact of different natural zeolite concentrations on the methane production in thermophilic anaérobie digestion of pig waste », Biosystemes Engineering, 99, 2008, 105-111, Kotsopoulos et al. ont étudié l'effet de la zéolite sur la méthanisation du lisier de porc en conditions thermophiles et ont montré une augmentation de la production du biogaz.

Dans « Application of natural zeolites in anaérobie digestion processes: A review », Applied Clay Sicence, 58, 2012, 125-133, Montalvo et al. ont également démontré que l'apport de la zéolite à la dose de 1 g/l lors du processus de méthanisation appliqué à des déjections porcines augmentait la dégradabilité de la matière organique et la production de biogaz.

Enfin, dans « Mitigating ammonia inhibition of thermophilic anaérobie treatment of digested piggery wastewater: Use of pH réduction, zeolite, biomass and humic acid », Water Research, 46, 2012, 4339-4350, Ho et al. ont montré que l'ajout de zéolite à du lisier et des boues de station d'épuration à des doses de 10 à 20 g/l augmentait la production du biogaz.

L'ensemble de ces publications suggèrent que l'effet de la zéolite sur la production du biogaz au cours la méthanisation par voie humide pourrait être attribué à son rôle de support physique pour les micro-organismes dans des effluents liquides pauvres en matières solides ainsi qu'à un effet de levée d'inhibition de la méthanisation par l'ion ammonium qu'elle contient, notamment pour ces effluents riches en azote. Pour ce faire, la zéolite est ajoutée à l'effluent directement dans le méthaniseur.

En outre, dans « Effect of particle size and doses of zeolite addition on anaérobie digestion processes of synthetic and piggery wastes », Process Biochemistry, 40, 2005, 1475-1481, Montalvo et al. expliquent que plus le diamètre des particules de zéolite utilisées est important, plus la surface de contact est importante et meilleure est l'efficacité du procédé de méthanisation par voie humide.

Néanmoins, aucune de ces publications ne suggère que l'apport de zéolite pourrait se faire avant l'introduction de l'intrant dans le méthaniseur ni que les résultats obtenus pourraient être transposables à une méthanisation par voie sèche utilisant par exemple des intrants à base de fumier pailleux ou contenant une proportion importante de carbone ligneux. En effet, de tels intrants représentent eux-mêmes un support physique potentiel pour les micro-organismes. En outre, étant plus pauvres en azote que les lisiers, ces intrants sont moins susceptibles de provoquer une inhibition de la méthanisation via l'ammonium. En conséquence, l'ajout de zéolite sur ce type d'intrants ne semblait pas a priori pouvoir améliorer la production du biogaz lors de la méthanisation. De plus, rien dans ces publications n'aurait incité l'homme du métier à travailler avec une zéolite possédant une granulométrie fine plutôt qu'avec une zéolite possédant une granulométrie plus grossière.

Or, il a maintenant été trouvé, de façon tout à fait surprenante, un nouveau procédé de méthanisation par voie sèche comprenant un prétraitement des déchets par l'ajout d'un additif de type zéolite ayant une granulométrie particulière (100 µm à 1000 µm) qui permet d'augmenter significativement la production de méthane et d'améliorer la rentabilité économique du procédé.

La présente invention comme définie par les revendications en annexe, a donc pour objet un procédé de méthanisation par voie sèche comprenant les étapes suivantes :
a) prétraitement de l'intrant comprenant l'ajout d'un additif choisi parmi la zéolite, l'argile, la pouzzolane et le biochar dont la granulométrie est comprise entre 100 µm et 1000 µm suivi ou précédé d'une hydrolyse ;
b) digestion anaérobie ;
c) séparation de la phase solide et de la phase liquide du digestat.

De façon tout à fait surprenante, le procédé selon la présente invention permet d'augmenter significativement la production de méthane en comparaison des procédés classiquement utilisés, tout en améliorant leur rentabilité économique.

Dans le cadre de la présente invention :
- on entend par « procédé de méthanisation par voie sèche » tout procédé conduit sur des intrants (également désignés par substrats, déchets ou effluents) possédant un taux de matière sèche supérieur ou égal à 20% en entrée du procédé permettant la production de biogaz, notamment de méthane, et d'un digestat ;
- on entend par « intrant », « déchet » ou « effluent » les matières organiques à pouvoir méthanogène utilisées pour la charge de l'installation de méthanisation, telles que les lisiers ou fumiers, les matières végétales, les matières organiques ou les déchets de végétaux de matières organiques (provenant de l'agriculture, de l'industrie agroalimentaire, de la restauration), de boues ou d'effluents de stations d'épuration;
- on entend par « digestat » toute substance résiduelle sous forme liquide, pâteuse ou solide présente dans le digesteur à l'issue du processus de méthanisation et généralement constituée de bactéries excédentaires, de matières organiques non dégradées, de matières minéralisées et d'eau ;
- on entend par « matière sèche » toute matière brute composée de matière minérale et organique séchée à 100°C jusqu'à poids constant ;
- on entend par « taux de matière sèche » le rapport p/p, exprimé en %, des matières après séchage (MS) / matières brutes avant séchage ;
- on entend par « zéolite » toute zéolite connue de l'homme de l'art, qu'elle soit naturelle ou synthétique. De préférence, le terme zéolite désigne une zéolite naturelle choisie parmi la famille des analcimes telle que l'analcime, la pollucite, la wairakite, la bellbergite, la bikitaite, la boggsite et la brewsterite ; la famille des chabazites telle que la chabazite, la willhendersonite, la cowlesite, la dachiardite, l'edingtonite, l'épistilbite, l'érionite, la faujasite, le ferrienite et l'herschelite ; la famille des gismondines telle que l'amicite, la garronite, la gismondine, la gobbinsite, la gmelinite, la gonnardite et goosecreekite ; la famille des harmotomes telle que l'harmotome, la phillipsite et la wellsite ; la famille des heulandites telle que clinoptilolite, la heulandite, la laumonite, la levyne, la mazzite, la merlinoite, la montesommaite, la mordenite et la maricopaite ; la famille des natrolites telle que la mésolite, la ntrolite, la scolécite, l'offretite, paranatrolite, paulingite et la perlialite ; la famille des stilbites telle que la barrérite, la stilbite, la stellerite, la thomsonite, tschernichite et la yugawaralite ; la dachiardite de sodium ; et la tétranatrolite. De préférence encore, le terme zéolite désigne la clinoptilolite, la chabazite, la phillipsite, le ferrierite, la mordénite ou l'érionite. De façon tout à fait préférée, le terme zéolite désigne la clinoptilolite ; et
- on entend par « argile » toute argile connue de l'homme du métier. De préférence, le terme argile désigne des phyllosilicates d'aluminium dont les feuillets sont constitués de couches d'octaèdres Al(OH)₆ et de couches de tétraèdres SiO₄ reliées par les atomes O et OH mis en commun et formées de particules fines de l'ordre du µm parmi lesquelles la kaolinite (1/1), l'illite (2/1), la smectite (2/1), la glauconie, la chlorite (2/1), la vermiculite (2/1) et les argiles fibreuses telles que la sépiolite et l'attapulgite (ou paligorskite). De préférence encore, le terme argile désigne les argiles fibreuses telles que la sépiolite et l'attapulgite. De façon tout à fait préférée, le terme argile désigne la sépiolite.

Dans le cadre de la présente invention, l'expression « additif dont la granulométrie varie de 'x' µm à 'y' µm » désigne tout additif sous forme de poudre dont au moins 50% (en volume) des particules ont un diamètre compris entre 'x' µm et 'y' µm, le diamètre des particules pouvant être déterminé par toute méthode connue de l'homme du métier, notamment par tamisage.

De plus, dans le cadre de la présente invention, les proportions exprimées en % correspondent à des pourcentages massiques par rapport au poids total de l'entité considérée (par exemple la matière sèche).
La Figure 1 représente le schéma réactionnel du processus de méthanisation.
La Figure 2 représente une installation classique pour la mise en oeuvre du processus de méthanisation.
La Figure 3 représente les résultats expérimentaux obtenus lors de la mesure de production cumulée de biogaz en fonction de la granulométrie de la zéolite utilisée.
La Figure 4 représente les résultats expérimentaux obtenus lors de la mesure de production cumulée de biogaz en fonction de la granulométrie et de la concentration de zéolite utilisée dans le substrat.

L'étape a) du procédé selon la présente invention correspond donc à une étape de prétraitement de l'intrant comprenant notamment l'ajout d'un additif choisi parmi la zéolite, l'argile, la pouzzolane et le biochar dont la granulométrie varie de 100 µm à 1000 µm suivie ou précédée d'une hydrolyse.

De préférence, l'étape a) du procédé selon la présente invention est conduite dans les conditions suivantes, prises seules ou en combinaison :
- l'additif est choisi comme étant la zéolite ;
- la granulométrie de l'additif varie de 400 µm à 900 µm ; de préférence encore de 500 µm à 800 µm ;
- l'additif est ajouté dans des proportions variant de 5% à 15% en poids de matière sèche des intrants. De préférence encore, l'additif est ajouté dans des proportions variant de 6% à 14% en poids de matière sèche des intrants. De façon tout à fait préférée, l'additif est ajouté dans des proportions variant de 7% à 12% en poids de matière sèche des intrants;
- les intrants sont choisis parmi les fumiers pailleux (bovin, équins ou toute matière similaire à base de paille), les semences, le menu paille ou les végétaux bruts ;
- le taux de matière sèche de l'intrant varie de 20% à 90%, de préférence de 28% à 55% ;
- l'additif est ajouté à la matière première (intrant) et mélangé manuellement ou avec tout moyen approprié, permettant une répartition homogène de l'additif dans le substrat.
- le mélange de l'additif et des intrants est réalisé avant le début de la phase d'hydrolyse ;
- l'hydrolyse est réalisée par l'ajout d'eau et/ou de digestat liquide recyclé suite à l'étape de séparation de la phase solide et de la phase liquide du digestat (étape c) du procédé selon la présente invention - Figure 2) ;
- la température à laquelle l'ajout de l'additif et des intrants est réalisé varie de 20°C à 37°C ;
- le temps d'hydrolyse varie de 24h à 90h ; et/ou
- la température à laquelle l'hydrolyse est réalisée varie de 20°C à 55°C.

A l'issue de l'étape a) du procédé selon la présente invention, les intrants prétraités (ou substrats) sont injectés dans un digesteur afin de subir une digestion anaérobie (étape b)).

L'étape de digestion anaérobie peut être conduite par tout moyen connu de l'homme du métier, notamment par l'utilisation d'un digesteur vertical ou horizontal.

Deux régimes de température sont principalement utilisés : mésophile (de 30°C à 40°C) ou thermophile (de 45°C à 60°C).

L'étape b) de digestion anaérobie est réalisée en présence d'un inoculum ajouté au substrat, ledit inoculum pouvant être tout inoculum connu de l'homme du métier adapté à la nature de l'intrant utilisé et aux conditions de méthanisation.

De préférence, l'étape b) du procédé selon la présente invention est conduite dans les conditions suivantes, prises seules ou en combinaison :
- le digesteur est de type horizontal avec une alimentation de type piston ou de type « box », étanche, pourvu de systèmes d'égouttage, recirculation des « jus » par pompage et de gestion des gaz ;
- le régime de température est de type mésophile ou thermophile ;
- l'agitation est assurée par recirculation des jus ou par un arbre horizontal à palette dont la rotation est séquencée pour dégazer le substrat et faciliter l'avancement de la charge ; et/ou
- le maintien à température est assuré par un échangeur à plaques

A l'issue de l'étape b) du procédé selon la présente invention, on sépare la phase solide et la phase liquide du digestat obtenu (étape c) du procédé selon la présente invention).

La fonction principale de cette étape étant de séparer une fraction liquide peu chargée en matière sèche (MS) pour pouvoir amener les intrants à la bonne siccité et obtenir des digestats solides manipulables sans jus libres ou dont la structure facilite la circulation d'air pour en assurer le compostage ou éventuellement le séchage à l'air chauffé par récupération de chaleur d'installation de cogénération par exemple.

La phase solide et la phase liquide du mélange obtenu à l'étape b) peuvent être séparées par tout moyen connu de l'homme du métier. En particulier, on pourra utiliser un filtre presse ou procéder par séparation gravitaire et extraction de la phase liquide.

De préférence, les digestats en sortie de phase anaérobie sont directement introduits dans le séparateur sans prétraitement autre que celui propre aux particularités de la recette ou de l'installation.

A l'issue du procédé selon la présente invention, trois produits distincts sont obtenus :
- du biogaz constitué principalement de gaz carbonique (entre 30 et 50 %) et de méthane (entre 50 et 70 %) ; saturé en eau et contenant des traces d'impuretés gazeuses (NH₃, H₂S, N₂, CO);
- une phase liquide contenant de l'eau et des sels solubilisés et des fines particules de matière sèche (MS). Généralement le taux de matière sèche (MS) est inférieur à 7 % ; de préférence compris entre 3 et 5 % et sont appelés « jus »;
- et une phase solide constituée de matières sèches grossières, à forte teneur en matières organiques stables et contenant encore une majorité quantitative de « jus » absorbés ou adsorbés (généralement de 65 à 72%). Généralement, la phase solide est exempte de « jus » libre.

Les fractions solides ou liquides non recyclées contenant de la matière organique et des éléments nutritifs pour les plantes (principalement azote, phosphore et potassium) ont un statut de « déchets » et sont utilisables en plans d'épandage pour l'agriculture.

Les digestats obtenus présentent néanmoins des propriétés agronomiques certaines et sont susceptibles de supporter des transformations pour les enrichir, et faciliter leur stockage, leur manutention et leur dosage à l'application. La première transformation est généralement un séchage pour concentrer la matière organique et les éléments nutritifs en utilisant de la chaleur produite par la production d'électricité. Le produit ainsi séché peut être mélangé à d'autres matières fertilisantes et transformé en granules par exemple.

La présente invention est illustrée de façon non limitative par les exemples suivants.

### Exemple : Procédé de méthanisation selon l'invention

### A - Protocole expérimental

### 1.1 - Prétraitement (étape a))

### 1.1.1 - Intrant utilisé

L'intrant utilisé est constitué à 79% de fumier (fumier de cheval pailleux) et 21% de légumes frais (carottes, courgettes, haricots verts à parts sensiblement égales).

Les fumiers sont préalablement broyés pour obtenir des fibres éclatées d'une longueur maximum de 2 cm.

Les légumes sont quant à eux coupés pour que leur taille varie de 1 à 2 cm.

### 1.1.2 - Additif utilisé

L'additif utilisé est une zéolite naturelle de la classe clinoptilolite (société Aquavista Limited), nom de marque TerraSorbTM.

Le grade < 700 µm a été testé tel que fourni.

Le grade 1 à 3 mm a été broyé en utilisant un broyeur à marteaux classique, puis l'échantillon broyé a été tamisé, à sec, en utilisant un appareil de tamisage de laboratoire : tamis vibrant RUSSEL Finex Screen - VSIA - A14550, avec différents étages de tamis empilés de la maille la plus petite (rang inférieur) à la maille la plus grosse (rang supérieur), de manière à disposer de différentes granulométries séparées, identifiées.

Les tamis utilisés sont les suivants :

| **Repère** | **Maille** | **Marque** | **Référence** |
|---|---|---|---|
| 11 | 3.15mm | SAULAS | NFX 11 |
| 10 | 2.5mm | Prüfsieb | AFNOR 547037 |
| 9 | 1.25mm | Prüfsieb | DIN 4188 |
| 8 | 1mm | Prüfsieb | AFNOR NFX 11.501 |
| 7 | 800µm | Prüfsieb | AFNOR NFX 11.501 |
| 6 | 500µm | Prüfsieb | AFNOR NFX 11.504 |
| 5 | 400µm | Prüfsieb | AFNOR NFX 11.501 |
| 4 | 125µm | SAULAS | NFX 11.501 |
| 3 | 100µm | Prüfsieb | AFNOR 11955 |
| 2 | 50µm | SAULAS | AFNOR 18 |
| 1 | 40µm | SAULAS | AFNOR 17 |

Dans le cadre des essais qui ont été menés, différentes granulométries de zéolite ont été testées :

| **Zéolite utilisée** | **Référence de l'essai** |
|---|---|
| TerraSorb^{™} grade 1 à 3 mm : zéolite naturelle broyée, tamisée entre tamis repères 1 & 2 - Taille 40 µ à 50 µ | Z 40-50 µ |
| TerraSorb^{™} grade 1 à 3 mm : zéolite naturelle broyée, tamisée entre tamis repères 3 & 4 - Taille 100 µ à 125 µ | Z 100-125 µ |
| TerraSorb^{™} grade 1 à 3 mm : zéolite naturelle broyée, tamisée entre tamis repères 5 & 6 -Taille 400 µ à 500 µ | Z 400-500 µ |
| TerraSorb^{™} grade 1 à 3 mm : zéolite naturelle broyée, tamisée entre tamis repères 6 & 7 - Taille 500 µ à 800 µ | Z 500-800 µ |
| TerraSorb^{™} de grade < 700 µ: zéolite naturelle, produit commercial | Z<700 µ |
| TerraSorb^{™} grade 1 à 3 mm : zéolite naturelle broyée, tamisée entre tamis repères 8 &9 - Taille 1mm à 1,25 mm | Z 1-1,25 mm |
| TerraSorb^{™} grade 1 à 3 mm : zéolite naturelle broyée, tamisée entre tamis repères 10 & 11 - Taille 2,5 mm à 3,15 mm | Z 2,5-3,15 mm |

### 1.1.3 - Prétraitement

La zéolite est mélangée manuellement et progressivement au substrat pour assurer une répartition homogène. L'eau est ajoutée progressivement lors du mélange de l'additif et du substrat pour obtenir un substrat ajusté à environ 40 % de matière sèche.

La zéolite est ajoutée dans des proportions de 5%, 10% ou 15% de matière sèche suivant l'essai réalisé.

Le substrat obtenu est par la suite stocké à température ambiante pendant environ 32h avant mise en méthanisation. La phase d'hydrolyse s'opère pendant ce temps de stockage.

### 1.2 - Digestion anaérobie (étape b))

### 1.2.1 - Préparation de l'inoculum

L'inoculum utilisé est issu d'un pilote dédié à la production d'inoculum. Ce pilote est entretenu en méthanisation continue dans un système infiniment mélangé (MS environ 7 %) et maintenu à 54°C, avec une agitation régulière et quotidienne. Il est alimenté deux fois par semaine par un mélange de céréales, de déchets verts et de boues de STEP. Les proportions de ce mélange sont de 8 parts pour les boues, 1 part pour les déchets verts, 1 part pour les céréales, de manière à obtenir un substrat d'environ 7% de MS.

A chaque alimentation, l'agitation est arrêtée et un volume connu (de 5 à 15 %) du digestat est retiré et remplacé par un volume équivalent du substrat. Le digestat sortant constitue l'inoculum pour les essais de méthanisation.

Avant l'utilisation de l'inoculum, ce dernier est testé pour déterminer son niveau d'activité et ses capacités à dégrader la matière organique. Pour ce test, un volume connu de l'inoculum est mis en présence d'une quantité connue d'acétate et mis en incubation thermophile pendant une durée d'environ 48h. L'inoculum est considéré actif quand au moins 80 % de l'acétate introduit est dégradé.

### 1.2.2 - Digestion anaérobie

Les digesteurs utilisés sont des enceintes en PVC d'environ 1 litre ou 6 litres fabriquées spécifiquement pour la méthanisation. Chaque enceinte est munie d'un bouchon d'alimentation et d'un orifice de sortie de biogaz.

Le substrat obtenu à l'étape 1.1.3 précédente est ajouté dans le digesteur, puis inoculé avec l'inoculum obtenu à l'étape 1.2.1 précédente. La quantité d'inoculum ajoutée est d'environ 39% de la quantité totale de substrat présente dans le digesteur. De l'eau est ajoutée afin d'ajuster la teneur en matière sèche à environ 24%.

La température dans le digesteur est maintenue à 54°C à l'aide d'un bain-marie. Le mélange est agité manuellement et quotidiennement (jours ouvrés) pendant 2 à 3 minutes.

La quantité de biogaz produit est mesurée une fois par jour durant toute la durée de l'essai.

### B - Résultats expérimentaux obtenus et conclusions

### 2.1 - Essai 1 - Granulométrie de la zéolite

L'essai a été réalisé selon les étapes a) et b) décrites dans les exemples 1.1 et 1.2 précédents dans les conditions opératoires suivantes :

| | |
|---|---|
| Volume du réacteur utilisé (en I) | 1 |
| Quantité d'intrants (en g) | 83,3 (fumier de cheval : 61 g / légumes : 16.3 g / eau :6g) |
| Quantité de zéolite (% en poids de matière sèche des intrants) | 10% (soit 2.7 g) |
| Quantité d'inoculum (en g) | 56 |

Les zéolites Z 40-50 µ, Z<700 µ, Z 1-1,25 mm et Z 2,5-3,15 mm ont été utilisées.

La production cumulée de biogaz pour chacun de ces substrats a été mesurée durant 30 jours et comparée à celle d'un substrat « témoin » auquel aucune zéolite n'a été ajoutée.

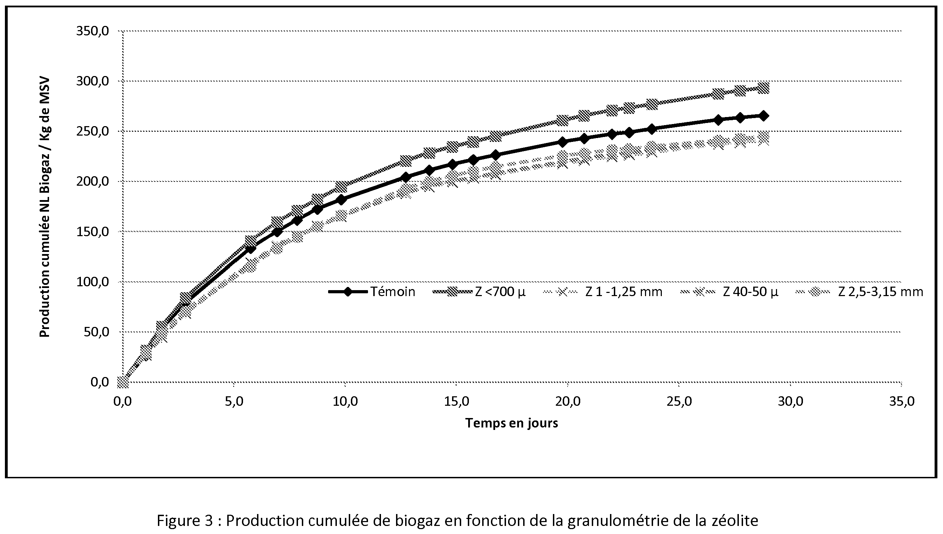

Les résultats obtenus sont rapportés dans la figure 3.

### 2.2 - Essai 2 - Granulométrie et concentration de la zéolite

L'essai a été réalisé selon les étapes a) et b) décrites dans les exemples 1.1 et 1.2 précédents dans les conditions opératoires suivantes :

| | |
|---|---|
| Volume du réacteur utilisé (en I) | 6.5 |
| Quantité de digestat (en g) | 520 (Fumier de cheval : 380g / légumes : 100 g / eau : 40g) |
| Quantité de zéolite (% en poids de matière sèche des intrants) | 10% (soit 21 g) |
| | ou |
| | 15 % (soit 31,5 g) |
| Quantité d'inoculum (en g) | 350 |

Les zéolites Z 100-125 µ, Z 400-500 µ, Z 500-800 µ et Z<700 µ ont été utilisées à différentes concentrations (10% ou 15% en poids de masse sèche des intrants).

La production cumulée de biogaz pour chacun de ces substrats a été mesurée durant 30 jours et comparée à celle d'un substrat « témoin » auquel aucune zéolite n'a été ajoutée.

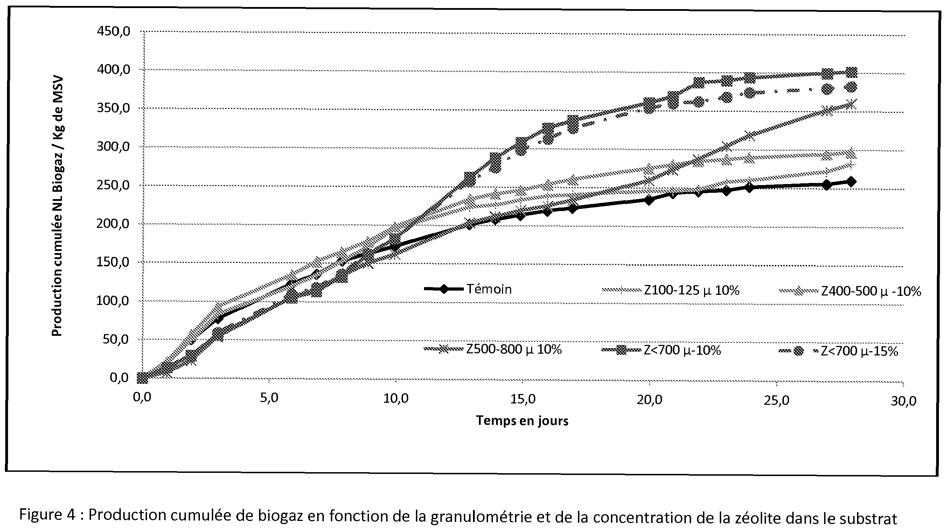

Les résultats obtenus sont rapportés dans la figure 4.

### 2.3 - Conclusions

L'apport d'une zéolite de granulométrie supérieure ou égale à 100 µm et inférieure ou égale à 1000 µm permet d'accélérer la production de biogaz d'une installation existante et donc d'augmenter la charge journalière en Matières Fraîches; ce qui se traduit par une augmentation d'énergie(s) produite(s), valorisable(s) dans la même proportion à installation et coûts fixes constants.

En outre, l'ajout de zéolite dans une proportion variant de 5% à 15% apparaît optimum en termes d'efficacité par rapport au coût.

## Revendications

1. Procédé de méthanisation par voie sèche comprenant les étapes suivantes :
a) prétraitement de l'intrant comprenant l'ajout d'un additif choisi parmi la zéolite, l'argile, la pouzzolane et le biochar dont la granulométrie varie de 100 µm à 1000 µm suivi ou précédé d'une hydrolyse ;
b) digestion anaérobie ;
c) séparation de la phase solide et de la phase liquide du digestat.

2. Procédé selon la revendication 1, **caractérisé en ce que** l'additif est de la zéolite.

3. Procédé selon la revendication 2, **caractérisé en ce que** la zéolite est choisie parmi la clinoptilolite, la chabazite, la phillipsite, le ferrierite, la mordénite et l'érionite.

4. Procédé selon la revendication 3, **caractérisé en ce que** la zéolite est de la clinoptilolite.

5. Procédé selon l'une quelconque des revendications 1 à 4, **caractérisé en ce que** la granulométrie de l'additif varie de 400 µm à 900 µm.

6. Procédé selon la revendication 5, **caractérisé en ce que** la granulométrie de l'additif varie de 500 µm à 800 µm.

7. Procédé selon l'une quelconque des revendications 1 à 6, **caractérisé en ce que** l'additif est ajouté dans des proportions variant de 5% à 15% en poids de matière sèche des intrants.

8. Procédé selon la revendication 7, **caractérisé en ce que** l'additif est ajouté dans des proportions variant de 6% à 14% en poids de matière sèche des intrants

9. Procédé selon l'une quelconque des revendications 1 à 8, **caractérisé en ce que** dans l'étape a), le prétraitement est effectué avant l'hydrolyse.

10. Procédé selon l'une quelconque des revendications 1 à 9, **caractérisé en ce que** l'étape b) est conduite dans des conditions mésophiles ou thermophiles.

## Patentansprüche

1. Trockenes Methanisierungsverfahren, umfassend die folgenden Schritte:
a) Vorbehandlung des Eintrags, umfassend die Zugabe eines Additivs, das aus Zeolith, Ton, Puzzolan und Biokohle ausgewählt ist, dessen Korngrößenverteilung von 100 µm bis 1000 µm variiert, der eine Hydrolyse folgt oder vorausgeht;
b) anaerobe Gärung;
c) Trennung der festen Phase und der flüssigen Phase des Gärrests.

2. Verfahren nach Anspruch 1, **dadurch gekennzeichnet, dass** das Additiv Zeolith ist.

3. Verfahren nach Anspruch 2, **dadurch gekennzeichnet, dass** der Zeolith aus Clinoptilolit, Chabazit, Phillipsit, Ferrierit, Mordenit und Erionit ausgewählt ist.

4. Verfahren nach Anspruch 3, **dadurch gekennzeichnet, dass** der Zeolith Clinoptilolit ist.

5. Verfahren nach einem der Ansprüche 1 bis 4, **dadurch gekennzeichnet, dass** die Korngrößenverteilung des Additivs von 400 µm bis 900 µm variiert.

6. Verfahren nach Anspruch 5, **dadurch gekennzeichnet, dass** die Korngrößenverteilung des Additivs von 500 µm bis 800 µm variiert.

7. Verfahren nach einem der Ansprüche 1 bis 6, **dadurch gekennzeichnet, dass** das Additiv in Anteilen zugegeben wird, die von 5 Gew.-% bis 15 Gew.-% Trockensubstanz der Einträge variieren.

8. Verfahren nach Anspruch 7, **dadurch gekennzeichnet, dass** das Additiv in Anteilen zugegeben wird, die von 6 Gew.-% bis 14 Gew.-% Trockensubstanz der Einträge variieren.

9. Verfahren nach einem der Ansprüche 1 bis 8, **dadurch gekennzeichnet, dass** die Vorbehandlung im Schritt a) vor der Hydrolyse vorgenommen wird.

10. Verfahren nach einem der Ansprüche 1 bis 9, **dadurch gekennzeichnet, dass** der Schritt b) unter mesophilen oder thermophilen Bedingungen durchgeführt wird.

## Claims

1. A dry methanation process comprising the following steps:
a) pre-treatment of the input comprising the addition of an additive selected from zeolite, clay, pozzolan and biochar, whose particle size varies from 100 µm to 1000 µm, followed or preceded by hydrolysis;
b) anaerobic digestion;
c) separation of the solid phase from the liquid phase of the digestate.

2. The process according to claim 1, **characterized in that** the additive is zeolite.

3. The process according to claim 2, **characterized in that** the zeolite is selected from clinoptilolite, chabazite, phillipsite, ferrierite, mordenite and erionite.

4. The process according to claim 3, **characterized in that** the zeolite is clinoptilolite.

5. The process according to any one of claims 1 to 4, **characterized in that** the particle size of the additive varies from 400 µm to 900 µm.

6. The process according to claim 5, **characterized in that** the particle size of the additive varies from 500 µm to 800 µm.

7. The process according to any one of claims 1 to 6, **characterized in that** the additive is added in proportions varying from 5% to 15% by weight of dry matter of the inputs.

8. The process according to claim 7, **characterized in that** the additive is added in proportions varying from 6% to 14% by weight of dry matter of the inputs.

9. The process according to any one of claims 1 to 8, **characterized in that** in step a), the pre-treatment is carried out before the hydrolysis.

10. The process according to any one of claims 1 to 9, **characterized in that** step b) is carried out under mesophilic or thermophilic conditions.
